# EUROPEAN PATENT SPECIFICATION

(11) **EP 1 602 320 B1**
(45) Date of publication and mention of the grant of the patent: **04.09.2013**
(21) Application number: 05011958.5
(22) Date of filing: 02.06.2005
(51) Int. Cl.: A61B 3/10

(54) **Ophthalmic apparatus**
Ophthalmologische Vorrichtung
Appareil ophthalmologique

(30) Priority: 03.06.2004 JP 2004165372; 08.06.2004 JP 2004169321
(43) Date of publication of application: 07.12.2005
(73) Proprietor: Nidek Co., Ltd., Gamagori-shi, Aichi-ken 443-0035 (JP)
(72) Inventor: Hanebuchi, Masaaki, Aichi-ken 444-0103 (JP)
(74) Representative: Winter, Brandl, Fürniss, Hübner, Röss, Kaiser, Polte - Partnerschaft

(56) References cited:
- WO-A-03/062802
- WO-A-2004/043245
- US-A- 5 975 699
- US-B1- 6 288 784
- PATENT ABSTRACTS OF JAPAN vol. 1996, no. 02, 29 February 1996 (1996-02-29) -& JP 07 255674 A (TOPCON CORP), 9 October 1995 (1995-10-09)

## Description

### BACKGROUND OF THE INVENTION

### 1. Field of the Invention

The present invention relates to an ophthalmic apparatus for obtaining ocular information.

### 2. Description of Related Art

Conventionally, on the basis of ocular information, various ophthalmological instruments such as an intraocular lens are designed and other various ophthalmological medical researches are carried out. As an example of an apparatus for obtaining the ocular information, there is an apparatus which performs noncontact measurement of eye refractive power and an axial length (see JP 7-255674). However, since the ocular information includes thickness of a cornea, thickness of a crystalline lens, depth of an anterior chamber, and the like in addition to the eye refractive power and the axial length, an apparatus capable of obtaining also these kinds of ocular information is desired.

US 6,288,784 B1 teaches an apparatus according to the preamble of claim 2.

### SUMMARY OF THE INVENTION

An object of the invention is to overcome the problems described above and to provide an ophthalmic apparatus individually capable of obtaining various kinds of ocular information.

To achieve the objects and in accordance with the purpose of the present invention, an ophthalmic apparatus as defined in claim 1 has a first projection optical system which projects first light with short coherence length onto a fundus of the examinee's eye, a second projection optical system which projects second light with short coherence length onto an anterior segment of the examinee's eye, an interference/photo-receiving optical system which synthesizes the first light reflected from the fundus and the second light reflected from the anterior segment into interference light, disperses the interference light into frequency components, and photo-receives the dispersed interference light with a first photodetector, and a calculation device for obtaining depth dimensions of the examinee's eye as the ocular information based on an output signal from the first photodetector.

Additional objects and advantages of the invention are set forth in the description which follows, are obvious from the description, or may be learned by practicing the invention. The objects and advantages of the invention may be realized and attained by the ophthalmic apparatus in the claims.

### BRIEF DESCRIPTION OF THE DRAWINGS

The accompanying drawings, which are incorporated in and constitute a part of this specification, illustrate embodiments of the present invention and, together with the description, serve to explain the objects and advantages of the invention. In the drawings,
Fig. 1 is a view showing a schematic configuration of an optical system of an ophthalmic apparatus consistent with the preferred embodiment of the present invention;
Fig. 2 is a schematic block diagram of a control system of the present apparatus;
Figs. 3A to 3C are views showing a convergence state of measurement light via a ring lens;
Fig. 4 is a view showing a schematic configuration of an optical system of an ophthalmic apparatus consistent with the second preferred embodiment of the present invention; and
Fig. 5 is a view showing a sectional image of an anterior segment of an eye displayed on a monitor.

### DETAILED DESCRIPTION OF THE PREFERRED EMBODIMENTS

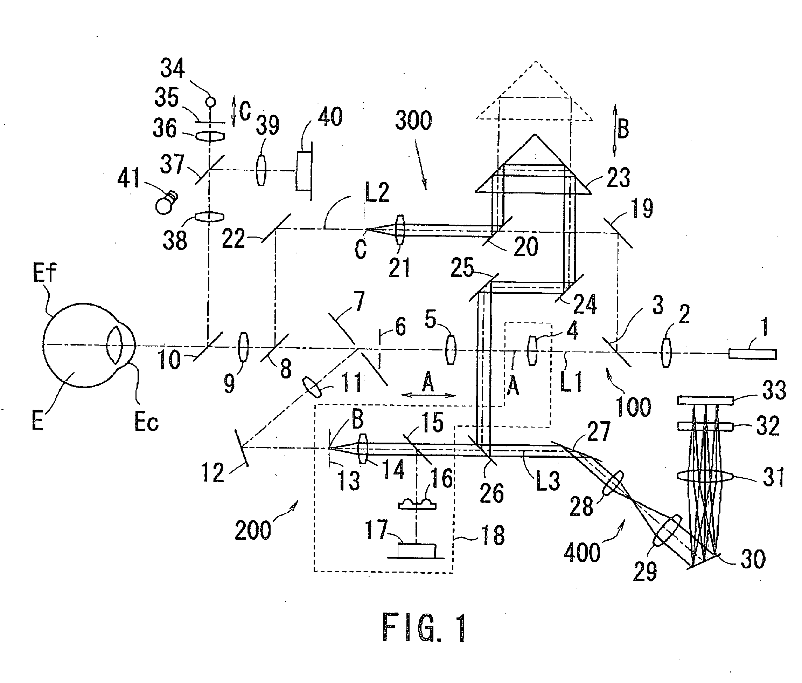

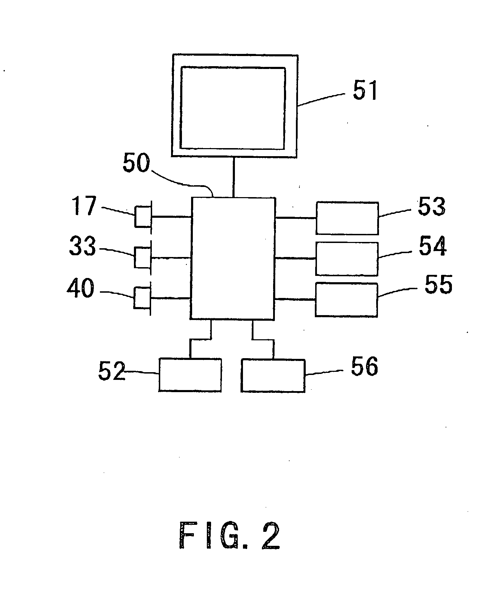

A detailed description of one preferred embodiment of an ophthalmic apparatus embodied by the present invention is provided below with reference to the accompanying drawings. Fig. 1 is a view showing a schematic configuration of an optical system of an ophthalmic apparatus consistent with the preferred embodiment of the present invention. Fig. 2 is a schematic block diagram of a control system of the present apparatus. The present apparatus is for obtaining ocular depth dimensions such as an axial length in addition to eye refractive power, and an optical system thereof includes a projection optical system for eye refractive power measurement and ocular depth dimension measurement, a photo-receiving optical system for eye refractive power measurement, a projection optical system for only ocular depth dimension measurement, an interference/photo-receiving optical system for ocular depth dimension measurement, and the like. Incidentally, while the present apparatus also has an optical system for aligning the apparatus with an examinee's eye to have a predetermined positional relationship therebetween, an optical system similar to a known optical system for alignment conventionally used in an objective eye refractive power measurement apparatus and the like may be used; therefore, description thereof is omitted.

### <Projection optical system 100 for eye refractive power measurement and ocular depth dimension measurement>

A projection optical system 100 includes a light source 1, a collimator lens 2, a beam splitter 3, a condenser lens 4, a relay lens 5, a diaphragm 6, a hole mirror 7, a beam splitter 8, an objective lens 9, and a beam splitter 10.

The light source 1 such as an infrared SLD (Super Luminescent Diode) emits infrared light with short coherence length. The light emitted from the light source 1 is made into parallel light by the collimator lens 2, and a part thereof passes through the beam splitter 3 to converge at a convergent point A on an optical axis L1 by the condenser lens 4, and passes through the relay lens 5, the diaphragm 6, and a hole (aperture) of the hole mirror 7 to converge, and passes through the beam splitter 8, the objective lens 9, and the beam splitter 10 to converge at a fundus Ef of an examinee's eye E.

### <Photo-receiving optical system 200 for eye refractive power measurement>

A photo-receiving optical system 200 includes the beam splitter 10, the objective lens 9, the beam splitter 8, the hole mirror 7 , a relay lens 11, a total reflection mirror 12, a diaphragm 13, a collimator lens 14, a beam splitter 15, a ring lens 16, a two-dimensional photodetector 17 having sensitivity to an infrared range. Incidentally, the photo-receiving optical system 200 shares the hole mirror 7 to the beam splitter 10 with the projection optical system 100.

Reflection light of the light converged at the fundus Ef by the projection optical system 100 passes through the beam splitter 10, the objective lens 9, and the beam splitter 8, and is reflected by a peripheral plane of the hole of the hole mirror 7. Incidentally, the hole mirror 7 reflects the reflection light from the fundus Ef and allows reflection light from a cornea Ec of the eye E to pass through its hole.

The fundus reflection light reflected by the hole mirror 7 passes through the relay lens 11, is reflected by the mirror 12 to converge at a convergent point B on the optical axis L1 and on an aperture plane of the diaphragm 13, and is made into parallel light by the collimator lens 14, and a part thereof is reflected by the beam splitter 15, passes through the ring lens 16, and is photo-received on the photodetector 17.

Incidentally, the convergent point A by the condenser lens 4 and the convergent point B at an anterior focal point of the collimator lens 14 are conjugate with the fundus Ef via the objective lens 9 and the relay lenses 5 and 11. In addition, the condenser lens 4, the diaphragm 13, the collimator lens 14, the beam splitter 15, the ring lens 16, and the photodetector 17 are arranged on a stage 18 movable in an optical axis direction (the arrow A direction in Fig. 1) by movement means 53.

In addition, the ring lens 16 is arranged at a posterior focal point of the collimator lens 14 and is conjugate with a pupil of the eye E regardless of a travel position of the stage 18 by the movement means 53. The photodetector 17 is arranged at a focal point of the ring lens 16 and is conjugate with the fundus Ef. Incidentally, the ring lens 16 is made of a plate on which a cylindrical lens is formed in a ring shape, and a light shielding coating is applied to a part other than the ring-shaped lens part. When the parallel light enters the ring lens 16, a ring image of approximately the same size as the ring lens 16 converges at the focal point (on a photo-receiving surface of the photodetector 17).

### <Projection optical system 300 for only ocular depth dimension measurement>

A projection optical system 300 includes the light source 1, the collimator lens 2, the beam splitter 3, a total reflection mirror 19, a beam splitter 20, a condenser lens 21, a total reflection mirror 22, the beam splitter 8, the objective lens 9, and the beam splitter 10. Incidentally, the projection optical system 300 shares the light source 1 to the beam splitter 3 and the beam splitter 8 to the beam splitter 10 with the projection optical system 100.

The light emitted from the light source 1 is made into parallel light by the collimator lens 2, and a part thereof is reflected by the beam splitter 3 to head for the mirror 19 arranged on an optical axis L2. The light reflected by the mirror 19 passes through the beam splitter 20 to converge at a convergent point C on the optical axis L2 by the condenser lens 21, is reflected by the mirror 22, is reflected by the beam splitter 8 to be coaxial with the optical axis L1, and passes through the objective lens 9 and the beam splitter 10 to converge at the cornea Ec.

### <Interference/photo-receiving optical system 400 for ocular depth dimension measurement>

An interference/photo-receiving optical system 400 is constituted of an optical system which photo-receives the reflection light from the fundus Ef and an optical system which photo-receives the reflection light from the cornea Ec.

The fundus reflection light photo-receiving optical system includes the beam splitter 10, the objective lens 9, the beam splitter 8, the hole mirror 7, the relay lens 11, the mirror 12, the diaphragm 13, the collimator lens 14, the beam splitter 15, a beam splitter 26, a total reflection mirror 27, a condenser lens 28, an expander lens 29, a grating mirror (diffraction grid) 30, a condenser lens 31, a cylindrical lens 32, and a one-dimensional photodetector 33 having sensitivity to the infrared range. Incidentally, the fundus reflection light photo-receiving optical system shares the beam splitter 10 to the beam splitter 15 with the photo-receiving optical system 200.

As for the reflection light of the light converged at the fundus Ef by the projection optical system 100, a part thereof passes through the beam splitter 15, further passes through the beam splitter 26 arranged on an optical axis L3, is reflected by the mirror 27, and passes through the condenser lens 28 to once converge. The fundus reflection light converged by the condenser lens 28 has its light bundle diameter enlarged by the expander lens 29, and is dispersed into frequency components by the grating mirror 30, and passes through the condenser lens 31 and the cylindrical lens 32 to converge at a photo-receiving surface of the photodetector 33.
Incidentally, the light bundle diameter after the passage through the expander lens 29, grid intervals and an angle of incidence for the grating mirror 30, the condenser lens 28, and the photodetector 33 are optimized in consideration of a measurement region in an optical axis direction and a resolution of the eye E.

On the other hand, the corneal reflection light photo-receiving optical system includes the beam splitter 10, the objective lens 9, the beam splitter 8, the mirror 22, the condenser lens 21, the beam splitter 20, a prism 23, total reflection mirrors 24 and 25, the beam splitter 26, the mirror 27, the condenser lens 28, the expander lens 29, the grating mirror 30, the condenser lens 31, the cylindrical lens 32, and the photodetector 33. The prism 23 is movable in the arrow B direction in Fig. 1 by movement means 54 and is capable of changing length of an optical path along which the corneal reflection light passes.

After the corneal reflection light passes through the beam splitter 10 and the objective lens 9, a part thereof is reflected by the beam splitter 8, is reflected by the mirror 22, passes through the condenser lens 21, and is reflected by the beam splitter 20 to head for the prism 23. The corneal reflection light reflected by the prism 23 is reflected by the mirrors 24 and 25 to head for the beam splitter 26. The corneal reflection light reflected by the beam splitter 26 is synthesized with the fundus reflection light. The corneal reflection light synthesized with the fundus reflection light goes via the mirror 27, the condenser lens 28, and the expander lens 29 as in the case of the fundus reflection light, and is dispersed into the frequency components by the grating mirror 30 and passes through the cylindrical lens 32 to converge at the photodetector 33. In this way, the grating mirror 30, the condenser lens 31, the cylindrical lens 32, and the photodetector 33 constitute a spectrometer part. Incidentally, the photo-receiving surface of the photodetector 33 is arranged to have a conjugate positional relationship with the fundus Ef and the cornea Ec. The cylindrical lens 32 acts to enlarge the light bundle diameter in a width direction of the photodetector 33 and is used to have the light photo-received on the photo-receiving surface regardless of a placement error of the photodetector 33.

### <Target projection optical system and observation optical system>

A target projection optical system shown in Fig. 1 includes a light source 34 such as a visible LED which emits visible light, a target plate 35 on which a predetermined target is formed, a relay lens 36, a beam splitter 37, an objective lens 38, and the beam splitter 10. The light emitted from the light source 34 illuminates the target plate 35 from behind. The light which passed through the target plate 35 passes through the relay lens 36, the beam splitter 37, the objective lens 38, and is reflected by the beam splitter 10 to form an image on the fundus Ef. Incidentally, when the apparatus is aligned with the eye E to form a predetermined positional relationship therebetween, the target plate 35 and the fundus Ef come to have a conjugate positional relationship. Besides, the light source 34 and the target plate 35 are movable in an optical axis direction (the arrow C direction in Fig. 1) by movement means 55 to perform fixation of the eye E and fog the eye E or apply accommodative load to the eye E by changing a position of the target plate 35 at the time of the eye refractive power measurement.

In addition, an observation optical system includes the beam splitter 10, the objective lens 38, the beam splitter 37, an image forming lens 39, and a two-dimensional photodetector 40 having sensitivity to the infrared range. The photodetector 40 is conjugate with the pupil. A light source 41 such as an infrared LED illuminates an anterior segment of the eye E.

A control part 50 controls to drive the apparatus. The control part 50 is connected with the photodetector 17, the photodetector 33, the photodetector 40, a monitor 51, a calculation/processing part 52, the movement means 53 to 55, a storage part 56, and the like. Each of the movement means 53 to 55 includes a pulse motor and the like, and a driving amount thereof is detectable. The calculation/processing part 52 is used to perform calculation for obtaining the ocular information such as the eye refractive power and the axial length of the eye E and form a sectional image of the anterior segment of the eye E based on information obtained by the photodetectors, the movement means, and the like. Calculated measurement values are stored in the storage part 56.

Hereinafter described will be operation of the apparatus with the configuration as stated above.

An examiner, while observing the monitor 51 shown in Fig. 2, moves the apparatus in up/down, right/left and back/forth directions by using unillustrated operating means such as a joystick to align the apparatus with the eye E so as to form a predetermined positional relationship therebetween. In this embodiment, a photo-receiving surface of the photodetector 40 and the pupil of the eye E are arranged to have a conjugate positional relationship. The examiner makes the target projected from the target projection optical system be fixated by the examinee and obtains the ocular information on the eye E by using an unillustrated measurement button.

When the measurement button is pressed, the control part 50 controls to drive the movement means 55 to move the light source 34 and the target plate 35 in the optical axis direction and bring a state where the eye E is fogged so as to eliminate its accommodation. In addition, the control part 50 controls the light source 1 to emit the light. The light emitted from the light source 1 is split into two directions by the beam splitter 3, and the respective light pass along the respective optical paths as mentioned above to converge at the fundus Ef and the cornea Ec, respectively.

The reflection light of the light converged at the fundus Ef is photo-received on the photodetector 17 via the aforementioned optical path.

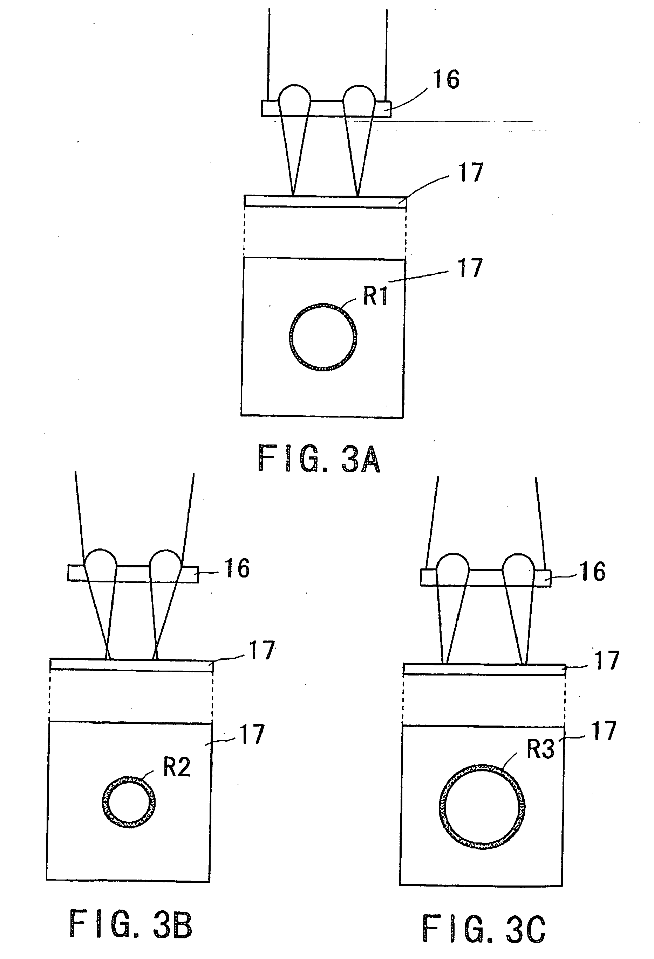

Figs. 3A to 3C are schematic diagrams showing a state where the fundus reflection light is photo-received on the photodetector 17 via the ring lens 16. For example, if the eye E is emmetropia, the fundus reflection light which passed through the collimator lens 14 becomes parallel light as shown in Fig. 3A to pass through the ring lens 16. In this case, a ring image R1 photo-received on the photodetector 17 via the ring lens 16 has approximately the same size with the ring part of the ring lens 16. Further, if the eye E is myopia, the fundus reflection light which passed through the collimator lens 14 becomes convergent light as shown in Fig. 3B to pass through the ring lens 16. In this case, a ring image R2 photo-received on the photodetector 17 via the ring lens 16 has a thicker rim and a smaller diameter than the ring image R1 shown in Fig. 3A. Furthermore, if the eye E is hyperopia, the fundus reflection light which passed through the collimator lens 14 becomes divergent light as shown in Fig. 3C to pass through the ring lens 16. In this case, a ring image R3 photo-received on the photodetector 17 via the ring lens 16 has a thicker rim and a larger diameter than the ring image R1 shown in Fig. 3A.

The calculation/processing part 52 performs calculation and processing based on an amount of change from a photo-receiving state in the case of the emmetropic eye to a photo-receiving state actually detected by the photodetector 17 to obtain eye refractive power of the eye E. In addition, based on a calculated value of the eyerefractive power or the photo-receiving statedetected by the photodetector 17, the calculation/processing part 52 calculates a position of the stage 18 with which the light projected toward the fundus Ef converges the most (in best focus) at the fundus Ef. The calculation/processing part 52 transmits information on the calculated position of the stage 18 where best focus is attained to the control part 50. The control part 50 drives the movement means 53 to move the stage 18 based on the received positional information and makes refractive power correction on the eye E. By making the refractive power correction on the eye E in this way, the convergence state at the fundus Ef of the light in accordance with the eye refractive power can be corrected, and an interference signal with a favorable S/N ratio can be obtained.

Incidentally, while the eye refractive power of the eye E is obtained based on the photo-receiving state of the ring image photo-received on the photodetector 17 in this embodiment, the present invention is not limited thereto. The eye refractive power of the eye E may also be obtained in consideration of the position of the stage 18 with which best focus is attained and the light converges at the fundus Ef, and the photo-receiving state of the ring image therewith. In addition, it is also possible to use the information on the photo-receiving state of the ring image photo-received on the photodetector 17 in calculating the position of the stage 18 for attaining best focus and obtain the eye refractive power of the eye E based on a travel amount of the stage 18 from a reference position. The obtained eye refractive power of the eye E is stored in the storage part 56 and displayed on the monitor 51.

Owing to the refractive power correction of the eye E by the movement of the stage 18, the light can efficiently converges at the fundus Ef. The fundus reflection light converges at the photodetector 33 via the aforementioned optical path, and the corneal reflection light converges at the photodetector 33 via the aforementioned optical path. As mentioned above, the synthetic light of the fundus reflection light and the corneal reflection light is dispersed into the frequency components by the grating mirror 30 and passes through the condenser lens 31 and the cylindrical lens 32 to converge at the photodetector 33. The photodetector 33 photo-receives the reflection light dispersed into the frequency components and outputs interference strength for each frequency component. The calculation/ processing part 52 monitors the interference strength obtained from the light photo-received on the photodetector 33.

On the other hand, the control part 50 controls the movement means 54 to move the prism 23 from a reference position indicated by solid lines (a position where the optical path becomes shortest is shown here) for changing the optical path length of the corneal reflection light. Incidentally, the prism 23 is given reciprocating motions several times to over ten times a second during the axial length measurement in this embodiment; however, the present invention is not limited thereto. The prism 23 may be moved simply in one direction instead of giving reciprocating motions. As this embodiment employs low coherent light, when the optical path of the fundus reflection light and that of the corneal reflection light become equal, strength of a signal of interference light obtained by synthesizing both of the light becomes maximum. Incidentally, the light photo-received on the photodetector 33 includes reflection light from phase objects such as a posterior surface of the cornea Ec and anterior/posterior surfaces of a crystalline lens in addition to the reflection light from an anterior surface of the cornea Ec. Accordingly, as the interference signal photo-received on the photodetector 33, these reflection light and the fundus reflection light are photo-received as a function of frequency.

The calculation/processing part 52 performs Fourier transform to analyze a detection signal outputted from the photodetector 33 when the strength of the interference signal becomes maximum. Since the interference light includes the reflection light from the respective phase objects of the eye E (e.g., the anterior/posterior surfaces of the cornea Ec, the anterior/posterior surfaces of the crystalline lens, and the fundus (a retina)), Fourier transform on the detection signal enables obtaining depth dimensions of the respective phase objects such as the cornea Ec and the crystalline lens of the eye E. The calculation/processing part 52 obtains the axial length of the eye E from positional information on the anterior surface of the cornea Ec with reference to the calculated position of the prism 23 and a travel amount of the prism 23 (or a driving amount of the movement means 54). In addition, from the obtained depth dimensions of the respective phase objects, the calculation/processing part 52 directly obtains other depth dimensions (e.g., thickness of the cornea, thickness of the crystalline lens, and depth of an anterior chamber). Information on the obtained depth dimensions is stored in the storage part 56 and displayed on the monitor 51. Incidentally, while an optical path length of the fundus reflection light and that of the corneal reflection light are made equal by changing the optical path length of the corneal reflection light in this embodiment, the present invention is not limited thereto. The optical path length of the fundus reflection light may be changed.

Incidentally, in a case where a more accurate axial length is to be obtained, an optical member for astigmatic correction constituted of a combination of a plurality of cylindrical lenses may be arranged at a conjugate position with the pupil between the collimator lens 14 and the condenser lens 27, or at an approximately conjugate position with the pupil between the objective lens 9 and the hole mirror 7. For example, a Stokes' cross-cylinder constituted of a combination of two cylindrical lenses, and the like can be used as the optical member for astigmatic correction. If the eye E has astigmatism, the ring image photo-received on the photodetector 17 via the ring lens 16 becomes oval, and astigmatic power and an astigmatic axial angle can be obtained through analysis of the photo-receiving state of the ring image by the calculation/processing part 42. Based on the obtained astigmatic power and astigmatic axial angle of the eye E, the aforementioned optical member for astigmatic correction is arranged so as to cancel out an astigmatic component of the eye E. Owing to such configuration, even if the eye E has the astigmatic component, the fundus reflection light efficiently converges at the photodetector 33, whereby measurement accuracy can be enhanced.

Incidentally, while the light by the projection optical system 300 is converged at the corneal surface in this embodiment, the present invention is not limited thereto. It is essential only that the reflection light from the phase objects of the eye E (the cornea, the crystalline lens, and the like) is photo-received on the photodetector in a state where the reflection light is dispersed into the frequency components, for example, a converging point of the light can be the pupil of the eye E.

Additionally, while the grating mirror (diffraction grid) is employed as dispersing means for dispersing the synthetic light of the fundus reflection light and the corneal reflection light into the respective frequency components, the present invention is not limited thereto. Some other dispersing means such as a prism and an acoustic optical element can be employed.

Further, while the ring image is formed on the photodetector in order to obtain the eye refractive power in this embodiment, the present invention is not limited thereto. It is essential only that the eye refractive power is obtained by detecting a state of change including positional change and shape change in the photo-received image formed on the photo-receiving surface without using interference. For example, the fundus reflection light may be divided into several dots via an optical member to converge at the photodetector, and the eye refractive power is obtained based on a photo-receiving state of a photo-received image thereof.

As mentioned above, the ophthalmic apparatus consistent with this embodiment is capable of obtaining the eye refractive power and the ocular depth dimensions such as the axial length at a time, and by further providing scanning means for scanning the light projected onto the eye E and by analyzing the output signal from the photodetector 33 according to the principle of spectral interference, a sectional image of the anterior segment of the eye E can be obtained.

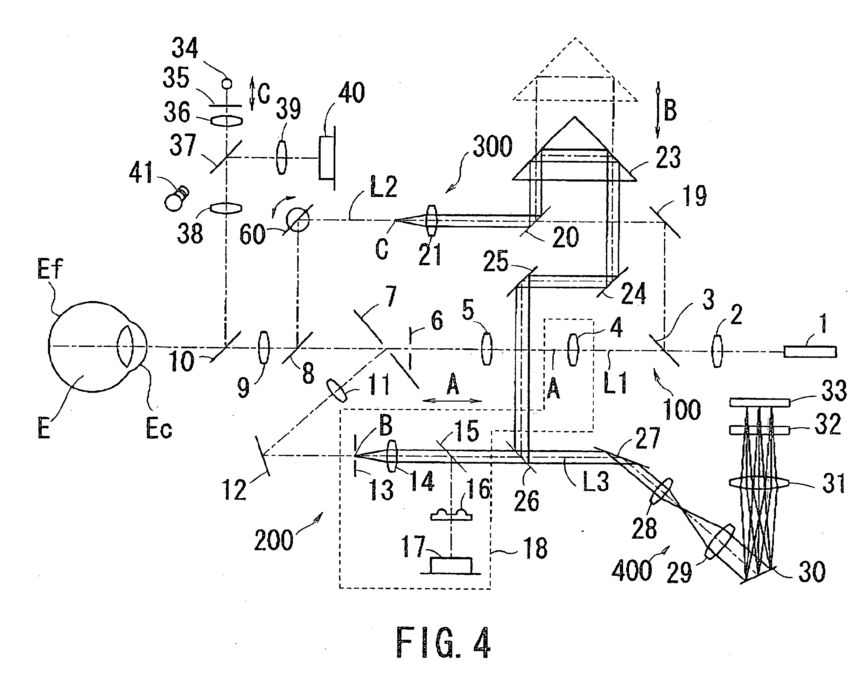

Fig. 4 is a view showing a schematic configuration of an optical system of an ophthalmic apparatus consistent with the second preferred embodiment of the present invention, in which the scanning means for scanning the light projected onto the eye E is provided to the optical system in Fig. 1. Incidentally, the constituting members of the same function as in Fig. 1 are assigned the same reference numerals, and description thereof is omitted.

A galvano mirror 60 is provided as the scanning means and can rotate (swing) in a predetermined direction (in this embodiment, a direction by which the light is scanned in the up/down directions with respect to the eye E). In addition, a reflection plane of the galvano mirror 60 is arranged at a focal point of the objective lens 9, and it is arranged such that a rotational angle of the galvano mirror 60 is proportional to a height of incidence of the light to the anterior segment of the eye E and the optical path length does not change. Since the control system of the present apparatus is the same as the control system shown in Fig. 2 except for driving and controlling of the galvano mirror 60, description thereof is omitted.

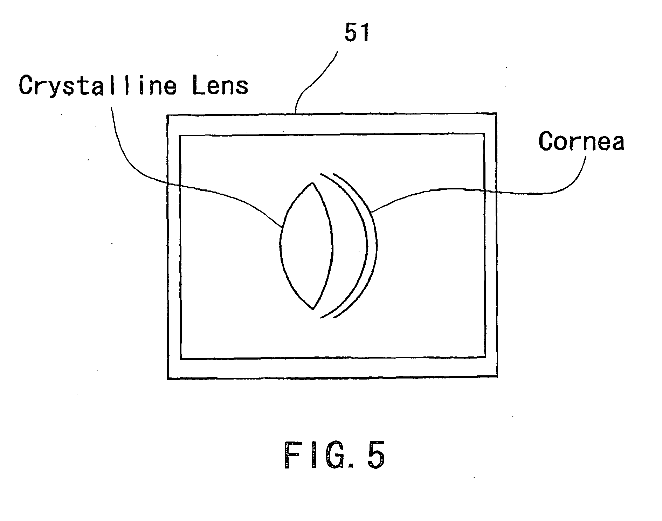

The light by the projection optical system 300 is scanned by the galvano mirror 60 in a predetermined direction with respect to the eye E. Accordingly, the output signal from the photodetector 33 includes information on the respective reflection light from the phase objects of the eye E obtained by scanning the light. By accumulating and adding the depth dimensions of the phase objects of the eye E which is obtained by means of Fourier transform on the output signal, the calculation/processing part 52 can obtain a sectional image of the anterior segment of the eye E which is optically sectioned by scanning. The obtained sectional image is displayed on the monitor 51 as shown in Fig. 5.

By using the reference light and the object light for interference and analyzing them according to the principle of spectral interference as mentioned above, both the reference light and object light change to the same degree even if the position of the eye E is somewhat deviated with respect to the apparatus. Therefore, the apparatus can obtain the depth dimensions and the sectional image of the eye E while withstanding alignment deviation and focus deviation.

The foregoing description of the preferred embodiments of the invention has been presented for purposes of illustration and description. It is not intended to be exhaustive or to limit the invention to the precise form disclosed, and modifications and variations are poss ible in the light of the above teachings. The embodiments chosen and described in order to explain the invention and its practical application to enable one skilled in the art to utilize the invention in various embodiments and with various modifications as are suited to the particular use contemplated. It is intended that the scope of the invention be defined by the claims appended hereto.

## Claims

1. An ophthalmic apparatus for obtaining ocular information on an examinee's eye (E), the apparatus comprising:
- a first projection optical system (100) which is configured to project first light with short coherence length onto a fundus (Ef) of the examinee's eye;
- a second projection optical system (300) which is configured to project second light with short coherence length onto an anterior segment of the examinee's eye;
- an interference/photo-receiving optical system (400) which is configured to synthesize the first light reflected from the fundus and the second light reflected from the anterior segment into interference light, to disperse the interference light into frequency components, and to photo-receive the dispersed interference light with a first photodetector (33); and
- calculation means (52) for obtaining depth dimensions of the examinee's eye as the ocular information based on an output signal from the first photodetector,
**characterized in that**:
- the interference/photo-receiving optical system includes optical path length changing means (23, 54) for changing an optical path length of the first light or the second light during eye axial length measurement, and
- the calculation means is configured to obtain an eye axial length of the examinee's eye based on an amount of change by the optical path length changing means and depth information on phase objects of the examinee's eye by performing Fourier transform on the output signal from the first photodetector.

2. The ophthalmic apparatus according to claim 1, wherein the calculation means is configured to obtain depth dimensions of the anterior segment based on the depth information.

3. The ophthalmic apparatus according to claim 1, further comprising a measurement optical system (100, 200) which is configured to project measurement light onto the fundus and to photo-receive the measurement light reflected from the fundus with a second photodetector (17), wherein the calculation means is configured to obtain eye refractive power of the examinee's eye as the ocular information based on an output signal from the second photodetector.

4. The ophthalmic apparatus according to claim 3, wherein the first projection optical system is shared as a part of the measurement optical system.

5. The ophthalmic apparatus according to claim 3, further comprising correction means (18, 53) for correcting a position of a part of the measurement optical system so as to adjust a convergence state of the measurement light at the fundus based on the obtained eye refractive power.

6. The ophthalmic apparatus according to claim 1, wherein
- the second projecting optical system includes scanning means (60) for scanning the second light, and
- the calculation means is configured to obtain a sectional image of the anterior segment as the ocular information based on a result of analyzing the output signal from the first photodetector by means of Fourier transform.

## Patentansprüche

1. Ophthalmologische Einrichtung zum Gewinnen einer Augeninformation über ein Auge (E) einer zu untersuchenden Person, wobei die Einrichtung umfasst:
- ein erstes optisches Projektionssystem (100), das dazu dient, ein erstes Licht mit kurzer Kohärenzlänge auf den Fundus (Ef) des Auges der zu untersuchenden Person zu projizieren;
- ein zweites optisches Projektionssystem (300), das dazu dient, ein zweites Licht mit kurzer Kohärenzlänge auf einen vorderen Abschnitt des Auges der zu untersuchenden Person zu projizieren;
- ein optisches Interferenz-/Lichtempfangssystem (400), das dazu dient, das von dem Fundus reflektierte erste Licht und das von dem vorderen Abschnitt reflektierte zweite Licht zu einem Interferenzlicht zu synthetisieren, das Interferenzlicht in Frequenzkomponenten aufzuspalten und das aufgespaltete Interferenzlicht mit einem ersten Fotodetektor (33) zu empfangen; und
- ein Berechnungsmittel (52) zum Gewinnen von Tiefendimensionen des Auges der zu untersuchenden Person als die Augeninformation auf der Grundlage eines Ausgangssignals von dem ersten Fotodetektor,
**dadurch gekennzeichnet, dass**:
- das optische Interferenz-/Lichtempfangssystem ein Mittel (23, 54) zum Ändern einer optischen Weglänge des ersten Lichts oder des zweiten Lichts während eines Messung der axialen Länge des Auges umfasst, und
- das Berechnungsmittel dazu dient, die axiale Länge des Auges der zu untersuchenden Person auf der Grundlage eines Änderungsbetrages durch das Mittel zum Ändern einer optischen Weglänge und einer Tiefeninformation über Phasenobjekte des Auges der zu untersuchenden Person mit Hilfe einer Fourier-Transformation des Ausgangssignals von dem ersten Fotodetektor zu gewinnen.

2. Ophthalmologische Einrichtung nach Anspruch 1, wobei das Berechnungsmittel dazu dient, Tiefendimensionen des vorderen Abschnitts auf der Grundlage der Tiefeninformation zu gewinnen.

3. Ophthalmologische Einrichtung nach Anspruch 1, ferner umfassend ein optisches Messsystem (100, 200), das dazu dient, ein Messlicht auf den Fundus zu projizieren und das von dem Fundus reflektierte Messlicht mit dem zweiten Fotodetektor (17) zu empfangen, wobei das Berechnungsmittel eine Augenbrechkraft des Auges der zu untersuchenden Person als die Augeninformation auf der Grundlage eines Ausgangssignals von dem zweiten Fotodetektor gewinnt.

4. Ophthalmologische Einrichtung nach Anspruch 3, wobei das erste optische Projektionssystem als ein Teil des optischen Messsystems geteilt wird.

5. Ophthalmologische Einrichtung nach Anspruch 3, ferner umfassend ein Korrekturmittel (18, 53) zum Korrigieren einer Position eines Teils des optischen Messsystems, um so einen Konvergenzzustand des Messlichts beim Fundus auf der Grundlage der gewonnenen Augenbrechkraft einzustellen.

6. Ophthalmologische Einrichtung nach Anspruch 1, wobei
- das zweite optische Projektionssystem ein Scanmittel (60) zum Scannen des zweiten Lichts umfasst, und
- das Berechnungsmittel dazu dient, ein Schnittbild des vorderen Abschnitts als die Augeninformation auf der Grundlage eines Ergebnisses der Analyse des Ausgangssignals von dem ersten Fotodetektor mit Hilfe der Fourier-Transformation zu gewinnen.

## Revendications

1. Appareil ophtalmique destiné à obtenir des informations oculaires sur l'oeil d'un patient (E), ledit appareil comprenant :
- un premier système optique de projection (100) qui est configuré pour projeter une première lumière ayant une courte longueur de cohérence sur un fond (Ef) de l'oeil du patient ;
- un second système optique de projection (300) qui est configuré pour projeter une seconde lumière ayant une longueur de cohérence courte sur un segment antérieur de l'oeil du patient ;
- un système optique d'interférence/de photo-réception (400) qui est configuré pour synthétiser ladite première lumière réfléchie par ledit fond et ladite seconde lumière réfléchie par ledit segment antérieur en une lumière d'interférence, pour disperser ladite lumière d'interférence en composantes de fréquence, et pour photo-recevoir ladite lumière d'interférence dispersée avec un premier photodétecteur (33) ; et
- un moyen de calcul (52) destiné à fournir les dimensions de profondeur de l'oeil du patient sous forme d'informations oculaires, sur la base d'un signal de sortie dudit premier photodétecteur,
**caractérisé en ce que** :
- ledit système optique d'interférence/de photo-réception comprend un moyen de changement de longueur de trajet optique (23, 54) destiné à changer une longueur de trajet optique de ladite première lumière ou de ladite seconde lumière pendant la mesure de la longueur axiale de l'oeil, et
- ledit moyen de calcul est configuré pour fournir une longueur axiale de l'oeil du patient sur la base d'une quantité de changement par ledit moyen de changement de longueur de trajet optique et d'informations de profondeur sur des objets de phase de l'oeil du patient en effectuant une transformée de Fourier sur le signal de sortie dudit premier photodétecteur.

2. Appareil ophtalmique selon la revendication 1, dans lequel ledit moyen de calcul est configuré pour fournir les dimensions de profondeur dudit segment antérieur sur la base desdites informations de profondeur.

3. Appareil ophtalmique selon la revendication 1, qui comprend en outre un système optique de mesure (100, 200) qui est configuré pour projeter une lumière de mesure sur ledit fond, et pour photo-recevoir ladite lumière de mesure réfléchie par ledit fond à l'aide d'un second photodétecteur (17), dans lequel ledit moyen de calcul est configuré pour fournir le pouvoir de réfraction de l'oeil du patient sous forme d'informations oculaires, sur la base du signal de sortie dudit second photodétecteur.

4. Appareil ophtalmique selon la revendication 3, dans lequel ledit premier système optique de projection est partagé dans le cadre dudit système optique de mesure.

5. Appareil ophtalmique selon la revendication 3, qui comprend en outre un moyen de correction (18, 53) destiné à corriger une position d'une partie dudit système optique de mesure de façon à ajuster un état de convergence de ladite lumière de mesure au niveau dudit fond, sur la base du pouvoir de réfraction de l'oeil obtenu.

6. Appareil ophtalmique selon la revendication 1, dans lequel
- ledit second système optique de projection comprend un moyen de balayage (60) destiné à balayer ladite seconde lumière, et
- ledit moyen de calcul est configuré pour fournir une image en coupe dudit segment antérieur sous forme d'informations oculaires, sur la base d'un résultat de l'analyse du signal de sortie dudit premier photodétecteur, à l'aide d'une transformée de Fourier.
